# EUROPEAN PATENT APPLICATION

(11) **EP 4 736 836 A1**
(43) Date of publication of application: **06.05.2026**
(21) Application number: 24832100.2
(22) Date of filing: 28.06.2024
(51) Int. Cl.: A61K 8/33, A61K 8/03, A61K 8/24, A61K 8/31, A61K 8/34, A61K 8/39, A61K 8/41, A61K 8/60, A61K 8/89, A61Q 19/10

(54) **TWO-LAYER SEPARATION TYPE COMPOSITION**

(30) Priority: 29.06.2023 JP 2023107271
(71) Applicant: Kao Corporation, Tokyo 103-8210 (JP)
(72) Inventor: YUAN, Xiaoli, Tokyo 103-8210 (JP)
(74) Representative: Hoffmann Eitle
(86) International application number: PCT/JP2024/023506
(87) International publication number: WO 2025/005242

(57) **Abstract**

The present invention relates to an oil-water two-layer separation type composition which becomes an emulsified state by shaking, and is not only clearly separated into oil and water after a lapse of given time but can favorably remove the mixed stains in skin pores. The present invention relates to a two-layer separation type composition comprising the following components (A), (B), (C), and (D), wherein pH of an aqueous layer at 25°C is 8.6 or higher and 12.5 or lower: (A) a non-ester oil agent which is in a liquid state at 25°C, (B) alkyl or alkenyl glyceryl ether or alkyl or alkenyl polyglyceryl ether having one or more linear or branched alkyl groups or alkenyl groups having 8 or more and 22 or less carbon atoms, and having one or more hydroxy groups, (C) a non-ester surfactant having HLB of 8 or more, and (D) water.

## Description

### Field of the Invention

The present invention relates to a two-layer separation type composition separated into an oil layer and an aqueous layer in a stationary state.

### Background of the Invention

The forms of makeup stain-removing cosmetics are known to have aqueous type, oil-based type, and emulsified type. Among them, the emulsified type is allegedly favorable from the viewpoint of achieving both of a makeup stain-removing effect and use impression. However, the emulsified type contains emulsifier and thickener for stabilizing the emulsified system and thus presents stickiness problems ascribable to these components.

Accordingly, two-layer separation type compositions separated into an oil layer and an aqueous layer in a stationary state have been developed. These two-layer separation type compositions need to become an emulsified state by shaking upon application and additionally to clearly become an oil-water separated state after a lapse of given time. A two-layer separation type composition comprising a surfactant selected from the group consisting of sugar fatty acid ester and alkyl polyglycoside having HLB of less than 8, branched alkane having 8 to 18 carbon atoms, and 0 to 4% by mass of a silicone oil (Patent Literature 1), and a two-layer separation type composition containing 0.06% by mass to 1.8% by mass of a polyol derivative (Patent Literature 2) have been reported as such oil-water two-layer separation type compositions.

(Patent Literature 1) JP-B-6486918
(Patent Literature 2) WO 2020/110608

### Summary of the Invention

The present invention provides a two-layer separation type composition comprising the following components (A), (B), (C), and (D), wherein pH of an aqueous layer at 25°C is 8.6 or higher and 12.5 or lower:
(A) a non-ester oil agent which is in a liquid state at 25°C,
(B) alkyl or alkenyl glyceryl ether or alkyl or alkenyl polyglyceryl ether having one or more linear or branched alkyl groups or alkenyl groups having 8 or more and 22 or less carbon atoms, and having one or more hydroxy groups,
(C) a non-ester surfactant having HLB of 8 or more, and
(D) water.

### Detailed Description of the Invention

Makeup stains such as foundations do not only exist in skin surface or sulcus cutis but also exist in skin pores. Stains in skin pores are often mixed stains of makeup cosmetics such as foundations and comedo sebum, and conventional makeup remover cosmetics have been found not to be able to favorably remove such stains in skin pores.

Thus, the present invention relates to providing an oil-water two-layer separation type composition which easily becomes an emulsified state by shaking, and not only clearly separates into oil and water after a lapse of given time but can favorably clean stains in skin pores.

The present inventor prepared two-layer separation type compositions by blending various components, and used the compositions to study not only the ease of emulsification and oil-water separability after emulsification but also a cleansing effect on mixed stains of foundation and comedo sebum in skin pores. As a result, the present inventor found that a composition excellent in the ease of emulsification, the oil-water separability, and the cleansing effect on stains in skin pores can be obtained by preparing a two-layer separation type composition using (A) a non-ester oil agent which is in a liquid state at 25°C, (B) alkyl or alkenyl glyceryl ether or alkyl or alkenyl polyglyceryl ether having a specific structure, (C) a non-ester surfactant having HLB of 8 or more, and (D) water, and adjusting pH of an aqueous layer at 25°C to 8.6 or higher and 12.5 or lower.

The two-layer separation type composition of the present invention easily becomes an emulsified state by shaking upon application, becomes an oil-water separated state after a lapse of given time, and can favorably cleanse not only makeup stains present in skin surface or sulcus cutis but mixed stains of a makeup cosmetic and comedo sebum present in skin pores.

The present invention relates to a two-layer separation type composition which easily becomes an emulsified state by shaking and becomes separated into an oil layer and an aqueous layer in a stationary state. One aspect is a two-layer separation type composition comprising the following components (A), (B), (C), and (D), wherein pH of an aqueous layer at 25°C is 8.6 or higher and 12.5 or lower:
(A) a non-ester oil agent which is in a liquid state at 25°C,
(B) alkyl or alkenyl glyceryl ether or alkyl or alkenyl polyglyceryl ether having one or more linear or branched alkyl groups or alkenyl groups having 8 or more and 22 or less carbon atoms, and having one or more hydroxy groups,
(C) a non-ester surfactant having HLB of 8 or more, and
(D) water.

The component (A) of the present invention is preferably a non-ester oil agent which is in a liquid state at 25°C from the viewpoint of oil-water separability and from the viewpoint of oil-water separability after preservation.

The non-ester oil agent which is in a liquid state at 25°C is preferably one or more members selected from the group consisting of a hydrocarbon oil, an ether oil, a silicone oil, and a higher alcohol. Among them, one or more members selected from the group consisting of a hydrocarbon oil, an ether oil, and a silicone oil are more preferred from the viewpoint of a cleansing effect on stains in skin pores.

The liquid state at 25°C refers to having flowability at 25°C. A state having a viscosity of 18 mPa·s or less at 25°C is more preferred from the viewpoint of oil-water separability. In this context, the viscosity is measured at 25°C using a B type viscometer (e.g., TVB-10 model manufactured by Toki Sangyo Co., Ltd., measurement conditions: rotor No. 1, 60 rpm, 1 min).

Examples of the hydrocarbon oil include liquid paraffin, squalane, light liquid isoparaffin, n-octane, n-heptane, cyclohexane, isododecane, and mineral oil. The hydrocarbon oil is preferably branched alkane having 8 or more and 22 or less carbon atoms, more preferably branched alkane having 8 or more and 18 or less carbon atoms, from the viewpoint of a cleansing effect on stains in skin pores.

Examples of the ether oil include cetyl dimethyl butyl, dicaprylyl ether, dilauryl ether, and diisostearyl ether.

Examples of the higher alcohol include liquid higher alcohols having 12 to 20 carbon atoms. A branched or unsaturated higher alcohol is preferred. Specific examples thereof include isostearyl alcohol, oleyl alcohol, and octyldodecanol.

Examples of the silicone oil include dimethylpolysiloxane (dimethicone), dimethyl cyclopolysiloxane, methylphenyl polysiloxane, methyl hydrogen polysiloxane, and higher alcohol-modified organopolysiloxane. It is preferred that the silicone oil contains at least dimethylpolysiloxane (dimethicone) from the viewpoint of a cleansing effect on the silicone oil in skin pores.

The content of the component (A) in the two-layer separation type composition is preferably 6% by mass or more, more preferably 7% by mass or more, further more preferably 14% by mass or more, even more preferably 20% by mass or more, and preferably 43% by mass or less, from the viewpoint of cleansing properties for stains in skin pores. The content is preferably 50% by mass or less, more preferably 34% by mass or less, from the viewpoint that the composition is emulsified for sufficient time for application to the skin by a given number of shake (hereinafter, referred to as the viewpoint of a favorable oil-water mixing time). More specifically, the content of the component (A) is preferably 6% by mass or more and 50% by mass or less, more preferably 7% by mass or more and 50% by mass or less, further more preferably 14% by mass or more and 43% by mass or less, even more preferably 20% by mass or more and 34% by mass or less, from the viewpoint of cleansing properties for stains in skin pores and a favorable oil-water mixing time.

The component (B) of the present invention is preferably alkyl or alkenyl glyceryl ether or alkyl or alkenyl polyglyceryl ether having one or more linear or branched alkyl groups or alkenyl groups having 8 or more and 22 or less carbon atoms, and having one or more hydroxy groups, from the viewpoint of oil-water separability. The component (B) preferably has HLB of less than 8. In this context, the HLB value is a value calculated by the Griffin method.

In this context, the alkyl group or the alkenyl group bonded through an ether bond to a glyceryl group or a polyglyceryl group is a linear or branched alkyl group or alkenyl group having 8 or more and 22 or less carbon atoms and is preferably a linear or branched alkyl group or alkenyl group having 8 or more and 18 or less carbon atoms from the viewpoint of oil-water separability.

At least one or more glyceryl groups or polyglyceryl groups are preferably hydroxy groups from the viewpoint of a cleansing effect on stains in skin pores.

The alkyl or alkenyl glyceryl ether and the alkyl or alkenyl polyglyceryl ether are preferably alkyl or alkenyl glyceryl ether having one or more linear or branched alkyl groups or alkenyl groups having 8 or more and 22 or less carbon atoms, and having one or more hydroxy groups from the viewpoint of oil-water separability and a cleansing effect on stains in skin pores, more preferably monoalkyl or monoalkenyl glyceryl ether having one linear or branched alkyl group or alkenyl group having 8 or more and 22 or less carbon atoms, further more preferably monoalkyl or monoalkenyl glyceryl ether having one branched alkyl group or alkenyl group having 8 or more and 18 or less carbon atoms from the viewpoint of oil-water separability. Specifically, isostearyl glyceryl ether (isostearyl glyceryl), stearyl glyceryl ether, isodecyl glyceryl ether, ethylhexylglycerin, cetyl glyceryl ether, oleyl glyceryl ether (also called selachyl alcohol), or the like is preferred, and isodecyl glyceryl ether is more preferred.

The content of the component (B) in the two-layer separation type composition is preferably 0.015% by mass or more, more preferably 0.03% by mass or more, further more preferably 0.036% by mass or more, even more preferably 0.05% by mass or more, from the viewpoint of a favorable oil-water mixing time and cleansing properties for stains in skin pores. The content is preferably 2% by mass or less, more preferably 1.5% by mass or less, further more preferably 0.09% by mass or less, from the viewpoint of oil-water separability and a cleansing effect on stains in skin pores. More specifically, the content of the component (B) is preferably 0.015% by mass or more and 2% by mass or less, more preferably 0.03% by mass or more and 1.5% by mass or less, further more preferably 0.036% by mass or more and 2% by mass or less, further more preferably 0.036% by mass or more and 1.5% by mass or less, even more preferably 0.05% by mass or more and 0.09% by mass or less, from the viewpoint of cleansing properties for stains in skin pores, oil-water separability, and a favorable oil-water mixing time.

The mass ratio of the component (B) to the component (A), (B/A), in the two-layer separation type composition is preferably 0.0065 or less, more preferably 0.0035 or less, further more preferably 0.0030 or less, even more preferably 0.0027 or less, from the viewpoint of oil-water separability.

The mass ratio of the component (B) to the component (A), (B/A), is preferably 0.0065 or less, more preferably 0.0035 or less, further more preferably 0.0030 or less, further more preferably 0.0027 or less, even more preferably 0.0015 or less, from the viewpoint of a cleansing effect on stains in skin pores. The mass ratio is preferably 0.0007 or more, more preferably 0.0008 or more, from the same viewpoint as above.

The mass ratio of the component (B) to the component (A), (B/A), is preferably 0.0007 or more, more preferably 0.0013 or more, further more preferably 0.0023 or more, from the viewpoint of a favorable oil-water mixing time.

The mass ratio of the component (B) to the component (A), (B/A), is preferably 0.0007 or more, more preferably 0.0008 or more, and preferably 0.0065 or less, more preferably 0.0035 or less, further more preferably 0.0030 or less, even more preferably 0.0027 or less, as a whole.

The component (C) of the present invention is preferably a non-ester surfactant having HLB of 8 or more from the viewpoint of oil-water separability and a favorable oil-water mixing time.

The component (C) is preferably one or more members selected from the group consisting of a nonionic surfactant, an anionic surfactant, and an amphoteric surfactant having HLB of from 8 to 18 from the viewpoint of oil-water separability and a favorable oil-water mixing time.

The HLB of the nonionic surfactant as the component (C) is preferably 8 or more and is more preferably 8.5 or more, further more preferably 9 or more, and preferably 18 or less, from the viewpoint of oil-water separability and a favorable oil-water mixing time.

Examples of the nonionic surfactant having HLB of from 8 to 18 include (C8-C20) alkyl polyglucoside (decyl glucoside and lauryl glucoside); polyoxyethylene alkyl ether such as polyoxyethylene lauryl ether (laureth-3, laureth-4, and laureth-6), polyoxyethylene oleyl ether, polyoxyethylene stearyl ether, polyoxyethylene behenyl ether, polyoxyethylene-2-octyl dodecyl ether, and polyoxyethylene cholestanol ether; polyoxyethylene polyoxypropylene alkyl ether such as polyoxyethylene polyoxypropylene cetyl ether, polyoxyethylene polyoxypropylene-2-decyl tetradecyl ether, polyoxyethylene polyoxypropylene monobutyl ether, polyoxyethylene polyoxypropylene hydrogenated lanoline, and polyoxyethylene polyoxypropylene glycerin ether; Pluronic type nonionic surfactants; tetrapolyoxyethylene tetrapolyoxypropylene-ethylenediamine condensates; and alkanolamide such as coconut oil fatty acid alkanolamide, lauric acid monoethanolamide, and fatty acid isopropanolamide. Among them, a (C8-C20) alkyl polyglucoside type nonionic surfactant having HLB of from 8 to 18 is preferred, and decyl glucoside or lauryl glucoside is more preferred.

Examples of the anionic surfactant include: higher fatty acid salts (e.g., sodium laurate and sodium palmitate); higher alkyl sulfuric acid ester salts (e.g., sodium lauryl sulfate and potassium lauryl sulfate); alkyl ether sulfuric acid ester salts (e.g., POE-triethanolamine lauryl sulfate and POE-sodium lauryl sulfate); N-acylsarcosine salts (e.g., sodium lauroyl sarcosine); higher fatty acid amide sulfonate (e.g., N-stearoyl-N-methyltaurine sodium, N-myristoyl-N-methyltaurine sodium, coconut oil fatty acid methyltaurine sodium, and sodium lauryl methyltauride); phosphoric acid ester salts (POE-sodium oleyl ether phosphate and POE-stearyl ether phosphate); sulfosuccinate (e.g., sodium di-2-ethylhexylsulfosuccinate, sodium monolauroyl monoethanolamide polyoxyethylene sulfosuccinate, and sodium lauryl polypropylene glycol sulfosuccinate); alkylbenzene sulfonate (e.g., sodium linear dodecylbenzenesulfonate, triethanolamine linear dodecylbenzenesulfonate, and linear dodecylbenzenesulfonate); higher fatty acid ester sulfuric acid ester salts (e.g., sodium hydrogenated coconut oil fatty acid glycerin sulfate); N-acyl glutamate (e.g., monosodium N-lauroyl glutamate (which is a component also called sodium lauroyl glutamate), disodium N-stearoyl glutamate, and monosodium N-myristoyl-L-glutamate); sulfated oils (e.g., sulfonated castor oil); POE-alkyl ether carboxylate; POE-alkyl allyl ether carboxylate; α-olefin sulfonate; higher fatty acid ester sulfonate; secondary alcohol sulfuric acid ester salts; higher fatty acid alkylolamide sulfuric acid ester salts; sodium lauroyl monoethanolamide succinate; ditriethanolamine N-palmitoyl aspartate; and casein sodium. Among them, an N-acylsarcosine salt, higher fatty acid amide sulfonate, sulfosuccinate, N-acyl glutamate (e.g., sodium lauroyl glutamate), polyoxyethylene alkyl ether carboxylate, or the like is preferred.

Examples of the amphoteric surfactant include imidazoline type amphoteric surfactants (e.g., 2-undecyl-N,N,N-(hydroxyethylcarboxymethyl)-2-imidazoline sodium and 2-cocoyl-2-imidazolinium hydroxide-1-carboxyethyloxy disodium salt); and betaine type surfactants (e.g., 2-heptadecyl-N-carboxymethyl-N-hydroxyethylimidazolinium betaine, lauryl dimethylaminoacetic acid betaine, alkyl betaine, amide betaine, and sulfobetaine (sultaine), (lauryl hydroxysultaine)).

The content of the component (C) in the two-layer separation type composition is preferably 0.001% by mass or more, more preferably 0.002% by mass or more, further more preferably 0.005% by mass or more, from the viewpoint of a favorable oil-water mixing time. The content is preferably 0.5% by mass or less, more preferably 0.3% by mass or less, further more preferably 0.049% by mass or less, from the viewpoint of oil-water separation. More specifically, the content of the component (C) is preferably 0.001% by mass or more and 0.5% by mass or less, more preferably 0.002% by mass or more and 0.3% by mass or less, further more preferably 0.005% by mass or more and 0.049% by mass or less, from the viewpoint of oil-water separability and a favorable oil-water mixing time.

The mass ratio of the component (B) to the component (C), (B/C), in the two-layer separation type composition is preferably 0.30 or more, more preferably 0.92 or more, from the viewpoint of oil-water separability.

The mass ratio of the component (B) to the component (C), (B/C), is preferably 750 or less, more preferably 45 or less, from the viewpoint of a cleansing effect on stains in skin pores.

The mass ratio of the component (B) to component (C), (B/C), is preferably 4 or more, more preferably 5 or more, further more preferably 7 or more, even more preferably 13 or more, particularly preferably 23 or more, when the amount of the component (C) is 0.004% by mass or less from the viewpoint of a favorable oil-water mixing time. The mass ratio of the component (B) to component (C) is preferably 25 or less, more preferably 8 or less, when the amount of the component (C) is larger than 0.004% by mass from the same viewpoint as above.

The mass ratio of the component (B) to the component (C), (B/C), is preferably 0.30 or more, more preferably 0.92 or more, and preferably 750 or less, more preferably 45 or less, as a whole.

The component (D) of the present invention is water.

Natural water, tap water, ion exchange water, pure water, or the like can be used as the water.

The content of the component (D) in the two-layer separation type composition is preferably 40% by mass or more and 93% by mass or less from the viewpoint of a favorable oil-water mixing time, oil-water separability, and use impression.

The pH of the aqueous layer at 25°C in the two-layer separation type composition of the present invention is preferably 8.6 or higher and 12.5 or lower from the viewpoint of improving cleansing properties for stains in skin pores. The pH is more preferably 8.7 or higher and 12.0 or lower, further more preferably 9.0 or higher and 11.5 or lower.

In order to adjust the pH of the aqueous layer to the range described above, a basic compound can be contained therein. It is preferred to contain one or more members selected from the group consisting of an amine compound, an inorganic salt group, and a water-soluble salt, and it is more preferred to use in combination an amine compound (E) and a water-soluble salt (F), for stably obtaining the advantageous effects of the present invention.

Examples of the amine compound (E) include: amino alcohol such as 2-amino-2-hydroxymethyl-1,3-propanediol (which is a component also called "tris(hydroxymethyl)aminomethane", "Tris", or "tromethamine"), 2-amino-2-methyl-1-propanol (which is a component also simply called "AMP"), 2-amino-2-methyl-1,3-propanediol (which is a component also simply called "AMPD"), monoethanolamine, and diethanolamine; chain or cyclic aliphatic amine such as methylamine, ethylamine, trimethylamine, triethylamine, ethylenediamine, tetramethylethylenediamine, hexamethylenediamine, diethylenetriamine, triethylenetetramine, tetraethylenepentamine, pentaethylenehexamine, and morpholine; and basic amino acids such as arginine and lysine. Among them, one or more members selected from the group consisting of 2-amino-2-hydroxymethyl-1,3-propanediol, 2-amino-2-methyl-1-propanol, 2-amino-2-methyl-1,3-propanediol, and arginine are more preferably used.

Examples of the water-soluble salt (F) include inorganic salts obtained from a strong base (potassium hydroxide, sodium hydroxide, lithium hydroxide, or the like) and a weak acid (carbonic acid, bicarbonic acid, acetic acid, phosphoric acid, citric acid, fatty acid, or the like), and inorganic salts obtained from the strong base and a strong acid (hydrochloric acid, sulfuric acid, or the like). Examples of the inorganic salt obtained from the strong base and the weak acid include potassium dihydrogen phosphate (potassium phosphate), dipotassium hydrogen phosphate, disodium hydrogen phosphate (disodium phosphate), trisodium citrate, sodium bicarbonate, sodium acetate, lithium carbonate, sodium carbonate, potassium carbonate, potassium bicarbonate, and potassium acetate. Examples of the inorganic salt obtained from the strong base and the strong acid include sodium chloride, potassium chloride, calcium chloride, magnesium chloride, sodium sulfate, potassium sulfate, magnesium sulfate, and calcium sulfate.

Such an amine compound or a water-soluble salt may be contained in an amount so that the pH of the aqueous layer at 25°C in the two-layer separation type composition of the present invention would fall within the range described above.

The mass ratio of the component (B) to the component (E), (B/E), in the two-layer separation type composition is preferably 0.0030 or more, more preferably 0.0054 or more, further more preferably 0.0090 or more, from the viewpoint of a favorable oil-water mixing time.

The mass ratio of the component (B) to the component (E), (B/E), is preferably 0.30 or less, more preferably 0.0020 or less, from the viewpoint of a cleansing effect on stains in skin pores.

The mass ratio of the component (B) to the component (E), (B/E), is preferably 0.0030 or more, more preferably 0.0054 or more, further more preferably 0.0090 or more, and preferably 0.30 or less, more preferably 0.0020 or less, as a whole.

The two-layer separation type composition of the present invention can contain, in addition to the components described above, an oil agent other than the component (A) (particularly, a skincare component such as a vegetable oil), a moisturizing agent such as polyol, an ultraviolet absorber, an antioxidant, a germicide, plant extracts (particularly, a skincare component such as a plant extract), a fragrance, a pigment, and the like without impairing the advantageous effects of the present invention. 0.05% by mass or less of the vegetable oil containing an ester oil may be contained.

The two-layer separation type composition of the present invention can be produced by mixing the components described above.

The obtained two-layer separation type composition of the present invention is a composition which easily becomes an emulsified state by shaking and becomes separated into an oil layer and an aqueous layer in a stationary state.

In this context, the composition is in an emulsified state for a sufficient time for application to the skin by a given number of shakes and offers favorable usability for application to the skin. The sufficient time for application of the composition to the skin can be 15 seconds or longer and is preferably 25 seconds or longer, more preferably 40 seconds or longer. In the present specification, this time is referred to as an oil-water mixing time.

The state "separated into an oil layer and an aqueous layer in a stationary state" means a state clearly separated into an oil layer and an aqueous layer. In the present specification, clear boundary between the oil layer and the aqueous layer in a stationary state is referred to as favorable oil-water separability.

Stains, such as makeup stains, present on the skin can be cleansed by cleansing the skin using the two-layer separation type composition of the present invention. In this context, stains present on the skin include not only makeup stains present in skin surface or sulcus cutis but mixed stains of a makeup cosmetic and comedo sebum present in skin pores. The comedo sebum refers to sebum stains present in skin pores. Use of the two-layer separation type composition of the present invention can cleanse both the makeup stains and the comedo sebum in skin pores, as a matter of course.

Thus, the two-layer separation type composition of the present invention is useful as a skin cosmetic and particularly useful as a composition for skin cleansing.

In order to clean the skin using the two-layer separation type composition of the present invention, the composition may be used to be washed off with water or may be used as leave-on type. The composition used as leave-on type may be applied to the skin and after massage, wiped off with a fiber material such as a pad, or the skin may be massaged with a fiber material impregnated with the composition.

In relation to the embodiments mentioned above, the present invention further discloses the following compositions, and the like.
<1> A two-layer separation type composition comprising the following components (A), (B), (C), and (D), wherein pH of an aqueous layer at 25°C is 8.6 or higher and 12.5 or lower:
   (A) a non-ester oil agent which is in a liquid state at 25°C,
   (B) alkyl or alkenyl glyceryl ether or alkyl or alkenyl polyglyceryl ether having one or more linear or branched alkyl groups or alkenyl groups having 8 or more and 22 or less carbon atoms, and having one or more hydroxy groups,
   (C) a non-ester surfactant having HLB of 8 or more, and
   (D) water.
<2> The two-layer separation type composition according to <1>, wherein the aqueous layer comprises an amine compound (E).
<3> The two-layer separation type composition according to <1> or <2>, wherein the aqueous layer comprises a water-soluble salt (F).
<4> The two-layer separation type composition according to any of <1> to <3>, wherein the component (A) is one or more members selected from the group consisting of a hydrocarbon oil, a higher alcohol, an ether oil, and a silicone oil which are in a liquid state at 25°C.
<5> The two-layer separation type composition according to any of <1> to <4>, wherein the component (A) is one or more members selected from the group consisting of branched alkane having 8 or more and 18 or less carbon atoms and a silicone oil.
<6> The two-layer separation type composition according to any of <1> to <5>, wherein a content of the component (A) is preferably 6% by mass or more and 50% by mass or less, more preferably 7% by mass or more and 50% by mass or less, further more preferably 14% by mass or more and 43% by mass or less, even more preferably 20% by mass or more and 34% by mass or less.
<7> The two-layer separation type composition according to any of <1> to <6>, wherein the component (B) is alkyl or alkenyl glyceryl ether having one or more linear or branched alkyl groups or alkenyl groups having 8 or more and 22 or less carbon atoms, and having one or more hydroxy groups.
<8> The two-layer separation type composition according to any of <1> to <7>, wherein the component (B) is monoalkyl or monoalkenyl glyceryl ether having one linear or branched alkyl group or alkenyl group having 8 or more and 22 or less carbon atoms.
<9> The two-layer separation type composition according to any of <1> to <8>, wherein a content of the component (B) is preferably 0.015% by mass or more and 2% by mass or less, more preferably 0.03% by mass or more and 1.5% by mass or less, further more preferably 0.05% by mass or more and 0.09% by mass or less.
<10> The two-layer separation type composition according to any of <1> to <9>, wherein the component (C) is one or more members selected from the group consisting of a nonionic surfactant, an anionic surfactant, and an amphoteric surfactant having HLB of 8 or more.
<11> The two-layer separation type composition according to any of <1> to <10>, wherein the component (C) is one or more members selected from the group consisting of decyl glucoside, lauryl glucoside, polyoxyethylene alkyl ether, polyoxyethylene polyoxypropylene alkyl ether, an N-acylsarcosine salt, higher fatty acid amide sulfonate, sulfosuccinate, N-acyl glutamate, polyoxyethylene alkyl ether carboxylate, an imidazoline type amphoteric surfactant, and a betaine type surfactant having HLB of from 8 to 18.
<12> The two-layer separation type composition according to any of <1> to <11>, wherein a content of the component (C) is preferably 0.001% by mass or more and 0.5% by mass or less, more preferably 0.002% by mass or more and 0.3% by mass or less, further more preferably, 0.005% by mass or more and 0.049% by mass or less.
<13> The two-layer separation type composition according to any of <1> to <12>, wherein the pH of the aqueous layer at 25°C is preferably 8.7 or higher and 12.0 or lower, more preferably 9.0 or higher and 11.5 or lower.
<14> The two-layer separation type composition according to any of <2> to <13>, wherein the amine compound is one or more members selected from the group consisting of amino alcohol, chain or cyclic aliphatic amine, and a basic amino acid.
<15> The two-layer separation type composition according to any of <2> to <14>, wherein the amine compound is one or more members selected from the group consisting of 2-amino-2-hydroxymethyl-1,3-propanediol, 2-amino-2-methyl-1-propanol, 2-amino-2-methyl-1,3-propanediol, monoethanolamine, diethanolamine, methylamine, ethylamine, trimethylamine, triethylamine, ethylenediamine, tetramethylethylenediamine, hexamethylenediamine, diethylenetriamine, triethylenetetramine, tetraethylenepentamine, pentaethylenehexamine, morpholine, arginine, and lysine.
<16> The two-layer separation type composition according to any of <2> to <15>, wherein the amine compound is one or more members selected from the group consisting of 2-amino-2-hydroxymethyl-1,3-propanediol, 2-amino-2-methyl-1-propanol, 2-amino-2-methyl-1,3-propanediol, and arginine.
<17> The two-layer separation type composition according to any of <3> to <16>, wherein the water-soluble salt is an inorganic salt comprising a strong base and a weak acid.
<18> The two-layer separation type composition according to any of <3> to <17>, wherein the water-soluble salt is one or more members selected from the group consisting of potassium dihydrogen phosphate (potassium phosphate), dipotassium hydrogen phosphate, disodium hydrogen phosphate (disodium phosphate), trisodium citrate, sodium bicarbonate, sodium acetate, lithium carbonate, sodium carbonate, potassium carbonate, potassium bicarbonate, and potassium acetate.
<19> The two-layer separation type composition according to any of <1> to <18>, wherein a mass ratio of the component (B) to the component (C), (B/C), is preferably 0.3 or more and 750 or less, more preferably 0.92 or more and 75 or less.
<20> The two-layer separation type composition according to any of <1> to <19>, wherein the two-layer separation type composition is a skin cosmetic.
<21> The two-layer separation type composition according to any of <1> to <20>, wherein the two-layer separation type composition is a composition for skin cleansing.

### Examples

Next, the present invention will be described in more detail with reference to Examples. However, the present invention is not limited by these Examples.

### Examples 1 to 43 and Comparative Examples 1 to 5

Components described in Tables 1 to 10 were mixed, and pH was adjusted to values described in these tables to produce two-layer separation type compositions. The obtained compositions were confirmed to be clearly separated into an oil layer and an aqueous layer in a stationary state.

The obtained compositions were evaluated for an oil-water mixing time, oil-water separability, and cleansing properties for stains in skin pores. The results are shown in Tables 1 to 10. Table 11 shows components used in a test.

### [1] Measurement of pH of aqueous layer

The pH of an undiluted solution of an aqueous layer at 25°C was measured with a pH meter (LAQUA F-72 from HORIBA, Ltd.).

### [2] Evaluation of oil-water mixing time

Each composition was shaken 10 times by hand with a stroke of approximately 30 cm, and a time that an emulsified state was maintained was measured. The oil-water mixing time was judged as being favorable when the time that the emulsified state was maintained was 15 seconds or longer, as being more favorable when this time was 25 seconds or longer, and as being particularly favorable when this time was 40 seconds or longer. The time (sec) that the emulsified state was maintained is shown in the tables.

### [3] Evaluation of oil-water separation

Each composition was emulsified by 10 shakes by hand with a stroke of approximately 30 cm and left standing for 1 day. Then, an oil-water interface was macroscopically checked and evaluated in accordance with scores given below. The compositions described in Table 3 were preserved at 60°C for 1 week and then checked for oil-water separability.
Score 1: The interface was white.
Score 2: Half the interface was white.
Score 3: The periphery of the interface was white.
Score 4: The interface was slightly white.
Score 5: The interface was uniformly transparent.

### [4] Evaluation of cleansing effect on stains in skin pore

10 mg of model comedo sebum described in Table 11 was applied onto artificial skin having skin pores (Okamoto Kaseihin Co., Ltd., regular white S2923), and 8 mg of a waterproof foundation (REVLON COLORSTAY 24hrs wear MOKA) was applied thereto 1 hour later. 0.1 g of a test composition was applied thereto 2 hours later and after massage 20 times, rinsed with tap water. Whether stains remained in the skin pores or not was observed under a microscope (KEYENCE VHX-5000, ×100 magnification) and evaluated in accordance with the following criteria. Score 1: Stains remained in almost all the pores of the artificial skin.
Score 1.5: Stains remained in more than half the pores of the artificial skin.
Score 2: Stains remained in about half the pores of the artificial skin.
Score 2.5: Stains remained in some of the pores of the artificial skin.
Score 3: Stains remained at the periphery of almost all the pores of the artificial skin.
Score 3.5: Stains remained at the periphery of some of the pores of the artificial skin.
Score 4: Stains slightly remained at the periphery of almost all the pores of the artificial skin.
Score 4.5: Stains slightly remained at the periphery of some of the pores of the artificial skin.
Score 5: No stains remained.

**[Table 1]**

| | | **Component name** | **Example 1** | **Example 2** | **Example 3** | **Example 4** |
|---|---|---|---|---|---|---|
| Oil layer | A | Isododecane | 20 | 20 | 20 | 20 |
| | B | Isodecyl glyceryl ether | 0.036 | 0.036 | 0.036 | 0.036 |
| Aqueous layer | C | Decyl glucoside | 0.002 | 0.002 | 0.002 | 0.002 |
| | E | Tromethamine | 5 | | | |
| | | AMP | | 5 | | |
| | | AMPD | | | 5 | |
| | | Arginine | | | | 5 |
| | | Potassium hydroxide | 0.29 | 0.29 | 0.29 | 0.29 |
| | F | Potassium phosphate | 1 | 1 | 1 | 1 |
| | D | Purified water | 73.67 | 73.67 | 73.67 | 73.67 |
| Total | | | 100 | 100 | 100 | 100 |
| | | | | | | |
| Evaluation item | pH | | 9.8 | 11.2 | 10.3 | 10.5 |
| | Oil-water mixing time/sec | | 25 | 25 | 25 | 24 |
| | Oil-water separation | | 5 | 5 | 5 | 5 |
| | Cleansing effect on stains in skin pore | | 4.5 | 5 | 3.5 | 4 |

**[Table 2]**

| | | **Component name** | **Example 1** | **Example 5** | **Example 6** | **Comparative Example 1** | **Comparative Example 2** | **Comparative Example 3** |
|---|---|---|---|---|---|---|---|---|
| Oil layer | A | Isododecane | 20 | 20 | 20 | 20 | 20 | 20 |
| | B | Isodecyl glyceryl ether | 0.036 | 0.036 | 0.036 | 0.036 | 0.036 | 0.036 |
| Aqueous layer | C | Decyl glucoside | 0.002 | 0.002 | 0.002 | 0.002 | 0.002 | 0.002 |
| | E | Tromethamine | 5 | 5 | 5 | 5 | 5 | 5 |
| | | Potassium hydroxide | 0.29 | | | | | |
| | F | Potassium phosphate | 1.0 | 1.0 | 1.5 | 2.3 | 4.5 | 5.9 |
| | D | Purified water | 73.67 | 73.96 | 73.46 | 72.66 | 70.46 | 69.06 |
| Total | | | 100 | 100 | 100 | 100 | 100 | 100 |
| | | | | | | | | |
| Evaluation item | pH | | 9.8 | 9.1 | 8.7 | 8.5 | 8.0 | 7.0 |
| | Oil-water mixing time/sec | | 25 | 30 | 25 | 29 | 35 | 30 |
| | Oil-water separation | | 5 | 5 | 5 | 5 | 5 | 5 |
| | Cleansing effect on stains in skin pore | | 4.5 | 4.5 | 3.5 | 2 | 1 | 1 |

**[Table 3]**

| | | **Component name** | **Example 1** | **Example 7** | **Example 8** | **Example 9** | **Comparative Example 4** |
|---|---|---|---|---|---|---|---|
| Oil layer | A | Isododecane | 20 | | | | |
| | | Mineral oil ^{*1} | | 20 | | | |
| | | Cetyl dimethyl butyl | | | 20 | | |
| | | Dimethicone ^{*2} | | | | 20 | |
| | | Isopropyl myristate | | | | | 20 |
| | B | Isodecyl glyceryl ether | 0.036 | 0.036 | 0.036 | 0.036 | 0.036 |
| Aqueous layer | C | Decyl glucoside | 0.002 | 0.002 | 0.002 | 0.002 | 0.002 |
| | E | Tromethamine | 5 | 5 | 5 | 5 | 5 |
| | | Potassium hydroxide | 0.29 | 0.29 | 0.29 | 0.29 | 0.29 |
| | F | Potassium phosphate | 1 | 1 | 1 | 1 | 1 |
| | D | Purified water | 73.67 | 73.67 | 73.67 | 73.67 | 73.67 |
| Total | | | 100 | 100 | 100 | 100 | 100 |
| | | | | | | | |
| Evaluation item | pH | | 9.8 | 9.8 | 9.8 | 9.8 | 9.8 |
| | Oil-water mixing time/sec | | 25 | 32 | 25 | 20 | 45 |
| | Oil-water separation | | 5 | 5 | 4 | 5 | 2 |
| | Oil-water separation (after preservation at 60°C for 1 week) | | 5 | 5 | 5 | 5 | 1 |
| | Cleansing effect on stains in skin pore | | 4.5 | 4 | 3.5 | 3.5 | 4 |

| | | | | | | | |
|---|---|---|---|---|---|---|---|
| ^{*}1 HICALL K-140N (Kaneda Co., Ltd.) ^{*}2 Silicone KF-96L-2CS (Shin-Etsu Chemical Co., Ltd.) | | | | | | | |

**[Table 4]**

| | | **Component name** | **Example 10** | **Example 11** | **Example 1** | **Example 12** | **Example 13** | **Example 14** |
|---|---|---|---|---|---|---|---|---|
| Oil layer | A | Isododecane | 7 | 10 | 20 | 30 | 40 | 50 |
| | B | Isodecyl glyceryl ether | 0.036 | 0.036 | 0.036 | 0.036 | 0.036 | 0.036 |
| Aqueous layer | C | Decyl glucoside | 0.002 | 0.002 | 0.002 | 0.002 | 0.002 | 0.002 |
| | E | Tromethamine | 5 | 5 | 5 | 5 | 5 | 5 |
| | | Potassium hydroxide | 0.29 | 0.29 | 0.29 | 0.29 | 0.29 | 0.29 |
| | F | Potassium phosphate | 1 | 1 | 1 | 1 | 1 | 1 |
| | D | Purified water | 86.67 | 83.67 | 73.67 | 63.67 | 53.67 | 43.67 |
| Total | | | 100 | 100 | 100 | 100 | 100 | 100 |
| | | | | | | | | |
| Evaluation item | pH | | 9.8 | 9.8 | 9.8 | 9.8 | 9.8 | 9.8 |
| | Oil-water mixing time/sec | | 30 | 30 | 25 | 30 | 20 | 20 |
| | Oil-water separation | | 4 | 4 | 5 | 5 | 5 | 5 |
| | Cleansing effect on stains in skin pore | | 3.5 | 3.5 | 4.5 | 5 | 5 | 4.5 |

**[Table 5]**

| | | **Component name** | **Example 1** | **Example 15** | **Example 16** | **Comparative Example 5** |
|---|---|---|---|---|---|---|
| Oil layer | A | Isododecane | 20 | 20 | 20 | 20 |
| | B | Isodecyl glyceryl ether | 0.036 | | | |
| | | Ethylhexylglycerin | | 0.036 | | |
| | | Isostearyl glyceryl | | | 0.025 | |
| | | Polyglyceryl-2 isostearate | | | | 0.040 |
| Aqueous layer | C | Decyl glucoside | 0.002 | 0.002 | 0.002 | 0.002 |
| | E | Tromethamine | 5 | 5 | 5 | 5 |
| | | Potassium hydroxide | 0.29 | 0.29 | 0.29 | 0.29 |
| | F | Potassium phosphate | 1 | 1 | 1 | 1 |
| | D | Purified water | 73.67 | 73.67 | 73.68 | 73.67 |
| Total | | | 100 | 100 | 100 | 100 |
| | | | | | | |
| Evaluation item | pH | | 9.8 | 9.8 | 9.8 | 9.8 |
| | Oil-water mixing time/sec | | 25 | 22 | 30 | 30 |
| | Oil-water separation | | 5 | 3 | 5 | 1 |
| | Cleansing effect on stains in skin pore | | 4.5 | 4 | 3 | 2.5 |

**[Table 6]**

| | | **Component name** | **Example 17** | **Example 1** | **Example 18** | **Example 19** | **Example 20** | **Example 21** | **Example 22** | **Example 23** | **Example 24** | **Example 25** |
|---|---|---|---|---|---|---|---|---|---|---|---|---|
| Oil layer | A | Isododecane | 20 | 20 | 20 | 20 | 20 | 20 | 20 | 20 | 20 | 20 |
| | B | Isodecyl glyceryl ether | 0.018 | 0.036 | 0.054 | 0.072 | 0.081 | 0.108 | 0.180 | 0.360 | 0.720 | 1.440 |
| Aqueous layer | C | Decyl glucoside | 0.002 | 0.002 | 0.002 | 0.002 | 0.002 | 0.002 | 0.002 | 0.002 | 0.002 | 0.002 |
| | E | Tromethamine | 5 | 5 | 5 | 5 | 5 | 5 | 5 | 5 | 5 | 5 |
| | | Potassium hydroxide | 0.29 | 0.29 | 0.29 | 0.29 | 0.29 | 0.29 | 0.29 | 0.29 | 0.29 | 0.29 |
| | F | Potassium phosphate | 1 | 1 | 1 | 1 | 1 | 1 | 1 | 1 | 1 | 1 |
| | D | Purified water | 73.69 | 73.67 | 73.65 | 73.64 | 73.63 | 73.60 | 73.53 | 73.35 | 72.99 | 72.27 |
| Total | | | 100 | 100 | 100 | 100 | 100 | 100 | 100 | 100 | 100 | 100 |
| | | | | | | | | | | | | |
| Evaluation item | pH | | 9.8 | 9.8 | 9.8 | 9.8 | 9.8 | 9.8 | 9.8 | 9.8 | 9.8 | 9.8 |
| | Oil-water mixing time/sec | | 15 | 25 | 40 | 40 | 50 | 50 | 44 | 44 | 44 | 44 |
| | Oil-water separation | | 5 | 5 | 5 | 5 | 5 | 5 | 5 | 5 | 5 | 5 |
| | Cleansing effect on stains in skin pore | | 4.5 | 4.5 | 4.5 | 4.5 | 4.5 | 4 | 4 | 4 | 4 | 4 |

**[Table 7]**

| | | **Component name** | **Example 26** | **Example 27** | **Example 28** | **Example 1** | **Example 29** | **Example 30** |
|---|---|---|---|---|---|---|---|---|
| Oil layer | A | Isododecane | 20 | 20 | 20 | 20 | 20 | 20 |
| | B | Isodecyl glyceryl ether | 0.036 | 0.036 | 0.036 | 0.036 | 0.036 | 0.036 |
| Aqueous layer | C | Laureth-3 | 0.002 | | | | | |
| | | Laureth-4 | | 0.002 | | | | |
| | | Laureth-6 | | | 0.002 | | | |
| | | Decyl glucoside | | | | 0.002 | | |
| | | Sodium lauroyl glutamate | | | | | 0.002 | |
| | | Lauryl hydroxysultaine | | | | | | 0.002 |
| | E | Tromethamine | 5 | 5 | 5 | 5 | 5 | 5 |
| | | Potassium hydroxide | 0.29 | 0.29 | 0.29 | 0.29 | 0.29 | 0.29 |
| | F | Potassium phosphate | 1 | 1 | 1 | 1 | 1 | 1 |
| | D | Purified water | 73.67 | 73.67 | 73.67 | 73.67 | 73.67 | 73.67 |
| Total | | | 100 | 100 | 100 | 100 | 100 | 100 |
| | | | | | | | | |
| Evaluation item | pH | | 9.8 | 9.8 | 9.8 | 9.8 | 9.8 | 9.8 |
| | Oil-water mixing time/sec | | 25 | 30 | 43 | 25 | 60 | 25 |
| | Oil-water separation | | 5 | 5 | 5 | 5 | 5 | 5 |
| | Cleansing effect on stains in skin pore | | 4 | 4 | 4 | 4.5 | 3.5 | 5 |

**[Table 8]**

| | | **Component name** | **Example 1** | **Example 31** | **Example 32** | **Example 33** | **Example 34** | **Example 35** | **Example 36** | **Example 37** |
|---|---|---|---|---|---|---|---|---|---|---|
| Oil layer | A | Isododecane | 20 | 20 | 20 | 20 | 20 | 20 | 20 | 20 |
| | B | Isodecyl glyceryl ether | 0.036 | 0.045 | 0.045 | 0.045 | 0.045 | 0.045 | 0.045 | 0.045 |
| Aqueous layer | C | Decyl glucoside | 0.002 | 0.004 | 0.008 | 0.016 | 0.024 | 0.048 | | |
| | | Sodium lauroyl glutamate | | | | | | | 0.050 | 0.100 |
| | E | Tromethamine | 5 | 5 | 5 | 5 | 5 | 5 | 5 | 5 |
| | | Potassium hydroxide | 0.29 | 0.29 | 0.29 | 0.29 | 0.29 | 0.29 | 0.29 | 0.29 |
| | F | Potassium phosphate | 1 | 1 | 1 | 1 | 1 | 1 | 1 | 1 |
| | D | Purified water | 73.67 | 73.66 | 73.66 | 73.65 | 73.64 | 73.62 | 73.62 | 73.57 |
| Total | | | 100 | 100 | 100 | 100 | 100 | 100 | 100 | 100 |
| | | | | | | | | | | |
| Evaluation item | pH | | 9.8 | 9.8 | 9.8 | 9.8 | 9.8 | 9.8 | 9.8 | 9.8 |
| | Oil-water mixing time/sec | | 25 | 30 | 60 | 110 | 120 | 120 | 60 | 110 |
| | Oil-water separation | | 5 | 5 | 5 | 5 | 5 | 5 | 4 | 4 |
| | Cleansing effect on stains in skin pore | | 4.5 | 4.5 | 4.5 | 4.5 | 4.5 | 4.5 | 4.5 | 4.5 |

**[Table 9]**

| | | **Component name** | **Example 1** | **Example 38** | **Example 39** | **Example 40** |
|---|---|---|---|---|---|---|
| Oil layer | A | Isododecane | 20 | 20 | 20 | 20 |
| | B | Isodecyl glyceryl ether | 0.036 | 0.036 | 0.036 | 0.036 |
| Aqueous layer | C | Decyl glucoside | 0.002 | 0.002 | 0.002 | 0.002 |
| | E | Tromethamine | 5 | 5 | 5 | 5 |
| | | Potassium hydroxide | 0.29 | | | |
| | F | Potassium phosphate | 1.00 | | | |
| | | Disodium phosphate | | 1.00 | | |
| | | Sodium chloride | | | 1.00 | |
| | | Sodium sulfate | | | | 1.00 |
| | D | Purified water | 73.67 | 73.96 | 73.96 | 73.96 |
| Total | | | 100 | 100 | 100 | 100 |
| | | | | | | |
| Evaluation item | pH | | 9.8 | 10.4 | 10.6 | 10.7 |
| | Oil-water mixing time/sec | | 25 | 30 | 25 | 25 |
| | Oil-water separation | | 5 | 5 | 5 | 5 |
| | Cleansing effect on stains in skin pore | | 4.5 | 4.5 | 4.5 | 4.5 |

**[Table 10]**

| | | **Component name** | **Example 41** | **Example 42** | **Example 43** |
|---|---|---|---|---|---|
| Oil layer | A | Isododecane | 20 | 20 | 20 |
| | B | Isodecyl glyceryl ether | 0.036 | 0.036 | 0.036 |
| Aqueous layer | C | Decyl glucoside | 0.002 | 0.002 | 0.002 |
| | E | Tromethamine | 5 | 5 | 5 |
| | F | Sodium chloride | 0.3 | 0.6 | 1.2 |
| | D | Purified water | 74.66 | 74.36 | 73.76 |
| Total | | | 100 | 100 | 100 |
| | | | | | |
| Evaluation item | pH | | 10.8 | 10.8 | 10.8 |
| | Oil-water mixing time/sec | | 24 | 22 | 22 |
| | Oil-water separation | | 5 | 5 | 5 |
| | Cleansing effect on dirt in skin pore | | 4.5 | 4.5 | 4.5 |

**[Table 11]**

| Composition of model comedo sebum | |
|---|---|
| **Chemical name** | **% by mass** |
| Lauric acid | 0.8 |
| Myristic acid | 6.4 |
| Palmitic acid | 24.0 |
| Oleic acid | 18.8 |
| Stearic acid | 4.8 |
| Myristyl myristate | 14.0 |
| Cholesterol ester (cholesterol palmitate) | 4.0 |
| Cottonseed oil | 7.2 |
| Squalene | 8.0 |
| Cholesterol | 12.0 |

As is evident from Tables 1 and 2, the cleansing properties for stains in skin pores are remarkably improved by adjusting the pH of the aqueous layer of each composition from 8.6 to 12.5. As is evident from Tables 3 and 4, the oil-water separability is improved by using the non-ester oil agent which is in a liquid state at 25°C as the oil agent. As is evident from Tables 5 and 6, the oil-water separability and the cleansing properties for stains in skin pores are improved by using the alkyl or alkenyl glyceryl ether or the alkyl or alkenyl polyglyceryl ether having one or more linear or branched alkyl groups or alkenyl groups having 8 or more and 22 or less carbon atoms, and having one or more hydroxy groups as the component (B). As is evident from Tables 7 and 8, the oil-water mixing time, the oil-water separability and the cleansing properties for stains in skin pores are improved by using the non-ester surfactant having HLB of 8 or more as the component (C). As is evident from Tables 9 and 10, the oil-water mixing time, the oil-water separability and the cleansing properties for stains in skin pores are improved by using the amine compound and the water-soluble salt as the pH adjuster.

## Claims

1. A two-layer separation type composition comprising the following components (A), (B), (C), and (D), wherein pH of an aqueous layer at 25°C is 8.6 or higher and 12.5 or lower:
(A) a non-ester oil agent which is in a liquid state at 25°C,
(B) alkyl or alkenyl glyceryl ether or alkyl or alkenyl polyglyceryl ether having one or more linear or branched alkyl groups or alkenyl groups having 8 or more and 22 or less carbon atoms, and having one or more hydroxy groups,
(C) a non-ester surfactant having HLB of 8 or more, and
(D) water.

2. The two-layer separation type composition according to claim 1, wherein the aqueous layer comprises an amine compound.

3. The two-layer separation type composition according to claim 1 or 2, wherein the aqueous layer comprises a water-soluble salt.

4. The two-layer separation type composition according to any one of claims 1 to 3, wherein the component (A) is one or more members selected from the group consisting of a hydrocarbon oil, a higher alcohol, an ether oil, and a silicone oil having a viscosity of 18 mPa·s or less at 25°C.

5. The two-layer separation type composition according to any one of claims 1 to 4, wherein the component (C) is one or more members selected from the group consisting of a nonionic surfactant, an anionic surfactant, and an amphoteric surfactant having HLB of 8 or more and 18 or less.

6. The two-layer separation type composition according to any one of claims 1 to 5, wherein a content of the component (A) is 6% by mass or more and 50% by mass or less, a content of the component (B) is 0.015% by mass or more and 2% by mass or less, and a content of the component (C) is 0.001% by mass or more and 0.5% by mass or less.

7. The two-layer separation type composition according to any one of claims 2 to 6, wherein the amine compound is one or more members selected from the group consisting of amino alcohol, chain or cyclic aliphatic amine, and a basic amino acid.

8. The two-layer separation type composition according to any one of claims 2 to 7, wherein the amine compound is one or more members selected from the group consisting of 2-amino-2-hydroxymethyl-1,3-propanediol, 2-amino-2-methyl-1-propanol, 2-amino-2-methyl-1,3-propanediol, monoethanolamine, diethanolamine, methylamine, ethylamine, trimethylamine, triethylamine, ethylenediamine, tetramethylethylenediamine, hexamethylenediamine, diethylenetriamine, triethylenetetramine, tetraethylenepentamine, pentaethylenehexamine, morpholine, arginine, and lysine.

9. The two-layer separation type composition according to any one of claims 2 to 8, wherein the amine compound is one or more members selected from the group consisting of 2-amino-2-hydroxymethyl-1,3-propanediol, 2-amino-2-methyl-1-propanol, 2-amino-2-methyl-1,3-propanediol, and arginine.

10. The two-layer separation type composition according to any one of claims 3 to 9, wherein the water-soluble salt is an inorganic salt comprising a strong base and a weak acid.

11. The two-layer separation type composition according to any one of claims 1 to 10, wherein the two-layer separation type composition is a skin cosmetic.

12. The two-layer separation type composition according to any one of claims 1 to 11, wherein the two-layer separation type composition is a composition for skin cleansing.
